# FASCICULE DE BREVET EUROPEEN

(11) **EP 1 112 499 B1**
(45) Date de publication et mention de la délivrance du brevet: **12.05.2004**
(21) Numéro de dépôt: 99942944.2
(22) Date de dépôt: 09.09.1999
(51) Int. Cl.: G01N 33/68, G01N 33/566, C07K 16/28

(54) **PROCEDE DE DETECTION OU DE QUANTIFICATION DES BASOPHILES ET DES EOSINOPHILES**
VERFAHREN ZUR ERFASSUNG ODER QUANTIFIZIERUNG VON BASOPHILEN UND EOSINOPHILEN
METHOD FOR DETECTING OR QUANTIFYING BASOPHILS AND EOSINOPHILS

(30) Priorité: 10.09.1998 FR 9811456
(43) Date de publication de la demande: 04.07.2001
(73) Titulaire: Immunotech, 13276 Marseille Cédex 9 (FR)
(72) Inventeur: MONTERO JULIAN Félix, A., 13008 Marseille (FR); MOREL MONTERO, Anne M., 13008 Marseille (FR); BRAILLY, Hervé, 13360 Roquevaire (FR); DELAAGE, Michel, 13001 Marseille (FR)
(74) Mandataire: Santarelli
(86) Numéro de dépôt international: PCT/FR1999/002145
(87) Numéro de publication internationale: WO 2000/016103

(56) Documents cités:
- EP-A- 0 811 691
- WO-A-97/48418
- US-A- 5 096 704
- SUN, QIYU ET AL: "Monoclonal antibody 7G3 recognizes the N-terminal domain of the human interleukin-3 (IL-3) receptor alpha-chain and functions as a specific IL-3 receptor antagonist." BLOOD, (1996) VOL. 87, NO. 1, PP. 83-92. ISSN: 0006-4971., XP002105606

## Description

La présente demande concerne un nouveau procédé de détection ou de quantification des basophiles et des éosinophiles chez des sujets sains ou malades, les procédés de préparation des réactifs nécessaires et leur mise en oeuvre notament sous forme de kits.

L'étude des éosinophiles et des basophiles est difficile à cause du très faible nombre de ces cellules dans le sang, soit, respectivement, de 1 à 3% et moins de 1%. Cependant, leur nombre peut augmenter de manière importante dans certains états pathologiques tels que les infections parasitaires, les états allergiques, asthmatiques ou certaines leucémies.

Il a été démontré que les éosinophiles jouent un rôle très important dans l'asthme allergique chronique. Les éosinophiles infiltrent les voies respiratoires où ils peuvent être activés et dégranuler. La libération du contenu granulaire, enzymes et protéines basiques, cause un dommage à l'épithélium bronchique. Certains rapports font aussi mention de dysfonctionnements des éosinophiles.

Le pontage des IgE spécifiques, présentes sur les basophiles, par différents allergènes, tels que les pneumallergènes, venins, protéines alimentaires ou médicaments peut entraîner la libération des médiateurs contenus dans les granules ou néoformés et capables d'induire un choc anaphylactique.

Il est donc important, pour connaître l'état d'un sujet et apprécier sa susceptibilité aux agents exogènes, de compter les éosinophiles et basophiles et de mesurer la proportion de ceux qui sont activables par tel ou tel agent.

Les méthodes de comptage des basophiles et des éosinophiles sont basées sur la coloration des granules (comptage manuel) avec, par exemple, du bleu d'ortho-toluidine pour les basophiles et de l'éosine pour les éosinophiles, sur leur taille et leur granulométrie ou sur un immunophénotypage différentiel de leur surface cellulaire. Ce sont des méthodes lourdes, consommatrices de temps, et peu spécifiques. Elles possèdent de grandes incertitudes et des erreurs statistiques importantes à cause du faible effectif de ces types cellulaires. D'autre part, la plupart de ces méthodes ne permet pas d'analyser le degré d'activation de ces cellules.

Pour toutes ces raisons, il serait souhaitable de disposer de produits et de procédés permettant de détecter et quantifier plus spécifiquement les éosinophiles et les basophiles, activés ou non.

Différents procédés, utilisant un cytomètre de flux, ont été décrits pour la détection des éosinophiles et des basophiles (voir par exemple Nakagawa, et al., (1981) Allergy, 36;pp. 39-47 ; Gane, et al., (1995) Cytometry 19;pp. 361-365 ; Hübl, et al., (1996) J. Clin. Lab. Analysis. 10, 177-183).

Un des principaux problèmes rencontrés lors de l'étude par cytométrie de flux des basophiles et des éosinophiles, est la séparation nette entre les cellules positives et les cellules négatives. Certains auteurs utilisent une caractéristique remarquable des éosinophiles, leur autofluorescence, pour les identifier plus précisément. Cependant, certains échantillons cliniques présentent une autofluorescence très élevée des monocytes, neutrophiles et lymphocytes. Il faut aussi souligner ici la grande variabilité de la granulométrie des éosinophiles, ce qui se traduit par un étalement de la zone du diagramme de dispersion (répartition en fonction de la taille et de la granulométrie) correspondant à ces cellules. Sur le diagramme de dispersion, les basophiles sont situés à la frontière entre les lymphocytes et les monocytes.

Les leucocytes expriment à leur surface des protéines caractérisées en CDs (Cluster Differenciation) par des groupes d'anticorps qui les reconnaissent. L'expression de plusieurs CDs sur une même cellule peut permettre de la caractériser. Un même CD peut être exprimé sur différents types cellulaires. C'est pourquoi la plupart des procédés par cytométrie de flux utilisent des mélanges d'anticorps reconnaissant plusieurs de ces CDs comme marqueurs des basophiles ou des éosinophiles.

Par exemple, l'utilisation de deux mélanges d'anticorps, anti-CD2, anti-CD14, anti-CD16 et anti-CD19 d'une part et anti-CD32, anti-CD25, anti-IgG1 et anti-IgG4 d'autre part, permet de détecter les basophiles au cytofluorimètre en flux. Les anticorps anti-CD2 mettent en évidence les cellules T, les anticorps anti-CD19 permettent de détecter les cellules B et les anticorps anti-CD14 détectent principalement les monocytes mais aussi les granulocytes. Le CD16, récepteur de type III des IgG, est exprimé sur les neutrophiles, certaines cellules T (NK) et sur les monocytes. Le CD32, récepteur de type II des IgG, est exprimé sur les monocytes, les neutrophiles et les lymphocytes B. Le CD25 est un marqueur d'activation des cellules T et B ainsi qu'un marqueur d'activation des macrophages. Plusieurs de ces marqueurs sont partagés par différents types de leucocytes. Les basophiles et les éosinophiles sont des populations minoritaires de leucocytes. C'est pourquoi la plupart des données sur les basophiles et les éosinophiles ont été obtenues à partir de cellules purifiées. Par exemple, les basophiles de sujets atteints de leucémie myéloïde chronique ont pu être étudiés après plusieurs étapes de purification à l'aide d'anticorps monoclonaux puis lyse des globules rouges par le complément. Cependant, ce procédé est impraticable en routine car il est long et nécessite un grand volume de sang.

D'autres procédés pour la détection des basophiles utilisent un anticorps anti-IgE ou un anticorps anti-récepteur de haute affinité pour les IgE. Mais ces procédés ne permettent pas une identification claire des basophiles car de faibles quantités de récepteur de haute affinité pour les IgE ont été mises en évidence sur les monocytes et les éosinophiles et les cellules B peuvent porter des IgE à leur surface. D'autre part, le pontage des IgE ou de leurs récepteurs à la surface des basophiles, par les anticorps utilisés comme sonde, peut entraîner une activation cellulaire modifiant les propriétés de la membrane. Il a été démontré que l'activation des basophiles par un allergène induit une diminution de la liaison d'un anticorps anti-IgE.

Il serait donc souhaitable de pouvoir facilement et sans risque d'erreur compter les éosinophiles et basophiles, même au milieu des autres populations cellulaires du sang humain et de mesurer la proportion de ceux qui sont activables par tel ou tel agent.

En réponse au problème du diagnostic et du traitement de maladies telles que l'asthme bronchique chronique ou la dermatite atopique, EP-A-0 811 691 propose des anticorps de divers types, monoclonaux, humanisés etc. dirigés contre la chaîne α du récepteur de l'interleukine-5 bloquant l'activité de cette cytokine. Il propose aussi un procédé de détection des éosinophiles. Mais l'utilisation d'anticorps neutralisant l'activité biologique de l'IL-5 donne des résultats décevants, par analyse en cytométrie de flux. Le signal spécifique obtenu sur des éosinophiles, même purifiés, ne se distingue pas du signal obtenu avec un anticorps de contrôle (bruit de fond).

Lors de la caractérisation d'un groupe d'anticorps monoclonaux dirigés contre le récepteur de l'interleukine-5 (RIL-5), la demanderesse a découvert que, de manière surprenante, les basophiles et les éosinophiles pouvaient, au milieu des autres populations cellulaires du sang humain ou animal, être détectés isolément et conjointement à l'aide de certains anticorps spécifiques de la chaîne alpha du récepteur de l'interleukine-5 et que l'on pouvait, si désiré, en plus compter ces mêmes cellules. La demanderesse a montré par bioessai sur cellules TF1, dépendantes de l'IL5 pour leur survie, que ces anticorps sélectionnés n'inhibaient pas la croissance de ces cellules.

D'autre part, la demanderesse a aussi montré que le récepteur lié à ces anticorps immobilisé sur phase solide est toujours capable de fixer l'IL5 marquée.

C'est pourquoi la présente demande a pour objet un procédé pour la détection ou la quantification des éosinophiles et des basophiles, caractérisé en ce qu'il comprend la mise en contact d'un échantillon contenant éventuellement lesdits éosinophiles ou basophiles avec un anticorps monoclonal anti-récepteur de l'IL-5 (chaîne alpha) qui n'interfère pas avec la fixation de l'IL5 à son récepteur et qui n'inhibe pas l'activité biologique de l'IL-5 pour détecter et si désiré procéder à la quantification des éosinophiles et des basophiles.

L'invention utilise donc un anticorps monoclonal anti-récepteur de l'IL-5 (chaîne alpha) et l'expression de la chaîne alpha du récepteur de l'IL-5 comme marqueur spécifique des éosinophiles et des basophiles. On a observé que ce marquage était spécifique de ces cellules sanguines et qu'aucune des cellules B, T, monocytes ou neutrophiles n'était marquée.

Ces anticorps sont donc utilisables pour le comptage des basophiles et des éosinophiles en toutes circonstances avec exactitude et précision.

L'échantillon contenant éventuellement lesdits éosinophiles ou basophiles peut être par exemple un prélèvement sanguin provenant notamment d'un sujet malade, de préférence allergique ou parasité.

Selon le procédé ci-dessus décrit, l'anticorps monoclonal anti-récepteur de l'IL-5 est un anticorps qui n'interfère pas avec la fixation de l'IL5 à son récepteur et qui n'inhibe pas l'activité biologique de l'IL-5.

L'absence d'inhibition de l'activité biologique de l'IL-5 peut être montrée par bioessai sur cellules TF1, dépendantes de l'IL5 pour leur survie, les anticorps selon l'invention n'inhibant pas la croissance de ces cellules TF1.

Dans des conditions préférentielles de mise en oeuvre du procédé ci-dessus décrit, l'anticorps monoclonal anti-récepteur de l'IL-5 est un anticorps qui n'interfère pas avec les IgE. L'on entend par «qui n'interfère pas avec les IgE» un anticorps qui n'empêche pas la liaison d'un allergène ou d'un autre anticorps anti-IgE, à ces IgE de surface .

Dans encore d'autres conditions préférentielles de mise en oeuvre du procédé ci-dessus décrit, l'anticorps monoclonal anti-récepteur de l'IL-5 est un anticorps qui n'interfère pas avec l'activation cellulaire des éosinophiles ou des basophiles. L'on entend par « qui n'interfère pas avec l'activation cellulaire des éosinophiles ou des basophiles» un anticorps qui par sa liaison à la surface de la cellule, n'induit pas ou n'inhibe pas l'apparition d'un marqueur d'activation de surface des basophiles ou des éosinophiles.

Dans toujours d'autres conditions préférentielles de mise en oeuvre du procédé ci-dessus décrit, la détection et si désiré la quantification des éosinophiles et des basophiles utilise un cytomètre de flux ou à balayage optique.

Les basophiles humains expriment à leur surface des marqueurs tels que les CD11, CD13, CD18, CD26, CD31, CD32, CD33, CD40, CD43, CD44, CD45, CD49d, CD54 et les éosinophiles expriment à leur surface les CD15, CD32, CD44, CD49d, CD52, CD65, CD66, CD67. Par contre ni les basophiles, ni les éosinophiles n'expriment les CD3, CD14, CD16, CD19. Ces listes de marqueurs ne sont pas exhaustives.

C'est pourquoi la présente demande a aussi pour objet un procédé ci-dessus caractérisé en ce que en outre l'on met en contact l'échantillon avec un ou de préférence plusieurs autres anticorps monoclonaux dirigés contre d'autres marqueurs permettant d'exclure les types cellulaires autres que les éosinophiles ou les basophiles. Ainsi on peut discriminer les lymphocytes, monocytes et neutrophiles des éosinophiles et basophiles.

On utilise donc des marqueurs d'exclusion pour améliorer encore la précision du comptage en excluant les événements erratiques. La présente demande a notamment pour objet un procédé ci-dessus caractérisé en ce que les autres anticorps monoclonaux sont dirigés contre les marqueurs CD3, CD16 et CD19 qui permettent d'exclure les événements erratiques.

L'invention permet la détection et la quantification spécifique des basophiles et des éosinophiles activés ou non activés. Il a été démontré récemment que le CD63, protéine lysosomale de la famille du tétraspan et initialement décrite comme marqueur d'activation des plaquettes, était également présent dans les granules des basophiles et neutrophiles. L'expression du CD63 à la surface cellulaire est dépendante du calcium. Lors de l'activation des basophiles, le CD63 exprimé à la surface peut être reconnu par un anticorps spécifique. L'intensité du marquage est fonction du nombre de cellules activées.

C'est pourquoi la présente demande a encore pour objet un procédé ci-dessus caractérisé en ce que l'on procède à la détection ou la quantification des basophiles activés en mettant en outre en contact l'échantillon avec un ou plusieurs autres anticorps monoclonaux dirigés contre des marqueurs d'activation des basophiles et notamment contre l'antigène CD 63.

De même, la présente demande a encore pour objet un procédé ci-dessus caractérisé en ce que l'on procède à la détection ou la quantification des éosinophiles activés en mettant en outre en contact l'échantillon avec un ou plusieurs autres anticorps monoclonaux dirigés contre des marqueurs d'activation des éosinophiles et notamment contre l'antigène CD 69.

De plus la détection et le comptage des basophiles et des éosinophiles séparément est possible dès lors que l'on dispose d'un moyen additionnel de discrimination tel que
- la diffusion de la lumière dans les cytomètres en flux où les éosinophiles se distinguent des basophiles par une valeur beaucoup plus élevée de la diffusion latérale (side scatter),
- l'activité peroxydasique propre aux éosinophiles que l'on révèle par utilisation de substrats précipitants comme le DAB (diamino benzidine) ou fluorescents comme le diacétate de dichlorofluorescéine, substrat non-fluorescent transformé en 2',7'-dichlorofluorescéine hautement fluorescente.

La présente demande a encore pour objet un anticorps anti-RIL-5 caractérisé par :
- l'absence d'interférence avec la fixation de l'IL5 à son récepteur
- l'absence d'interférence avec les IgE
- l'absence d'interférence avec l'activation cellulaire des éosinophiles ou des basophiles
- l'absence d'inhibition de l'activité biologique de l'IL-5.

Une réalisation avantageuse de l'invention consiste en l'utilisation d'un mélange d'anticorps dirigés d'une part contre le RIL-5, avec ou sans marqueurs "spécifiques" des lymphocytes T et B, cellules NK, monocytes et neutrophiles et, si l'on s'intéresse aux éosinophiles ou basophiles activés, d'autre part un marqueur d'activation de basophiles et des éosinophiles. Le marqueur d'activation cellulaire peut être un anticorps spécifique d'une protéine apparaissant à la surface de la membrane cellulaire après activation ou la mise en évidence d'une activité enzymatique oxydative. Celui-ci permet la mise en évidence et, éventuellement, la quantification de l'activation cellulaire.

C'est pourquoi la présente demande a aussi pour objet des kits ciblés pour différentes applications, lesdits kits ayant pour base un anticorps monoclonal anti-RIL-5, préférentiellement de souris, de rat ou de lapin, ou bien modifié génétiquement et premièrement
un kit pour la détection ou la quantification des éosinophiles ou des basophiles contenant
- au moins un anticorps monoclonal anti-RIL-5 tel que défini ci-dessus couplé à un premier fluorochrome,
- un mélange d'anticorps marqueurs des lymphocytes, monocytes et neutrophiles, couplés à un deuxième fluorochrome,
ainsi qu'un kit pour la détection et la quantification des éosinophiles et des basophiles activés contenant
- au moins un anticorps monoclonal anti-RIL-5 tel que défini ci-dessus couplé à un premier fluorochrome,
- un mélange d'anticorps marqueurs des lymphocytes, monocytes et neutrophiles, couplés à un deuxième fluorochrome et
- des anticorps dirigés contre des marqueurs d'activation et couplés à un troisième fluorochrome.

La présente demande a encore pour objet un kit ci-dessus caractérisé en ce qu'il renferme en outre un substrat spécifique de l'activité oxydative des éosinophiles.

La présente demande a aussi pour objet un kit pour la détection ou la quantification de l'activité oxydative des éosinophiles ou des basophiles contenant
- au moins un anticorps monoclonal anti-RIL-5 tel que défini ci-dessus couplé à un premier fluorochrome
- un mélange d'anticorps marqueurs des lymphocytes, monocytes et neutrophiles conjugués à un second fluorochrome
- un substrat marqueur de l'activité oxydative des éosinophiles ou des basophiles.

Les conditions préférentielles de mise en oeuvre des procédés ci-dessus décrites s'appliquent également aux autres objets de l'invention visés ci-dessus.

La présente demande a enfin pour objet un procédé, anticorps ou kit ci-dessus caractérisé en ce que l'anticorps monoclonal anti-récepteur de l'IL-5 est un anticorps de type IgG1 dont l'hybridome correspondant a été déposé à la Collection Nationale de Culture de Micro-organismes (CNCM) sous le N° I-2068.

L'invention peut être appliquée dans le test, par les laboratoires cliniques et pharmaceutiques, de la réponse des basophiles ou des éosinophiles à différents types d'agents dégranulants (allergènes, substances chimiques, parasites, etc.), afin de réaliser un diagnostic ou un suivi de désensibilisation, ou encore de mettre en évidence la capacité de nouvelles molécules à moduler la dégranulation des basophiles ou des éosinophiles et pour l'étude de pathologies allergiques, parasitaires et leucémiques.

Les exemples qui suivent illustrent la présente demande.

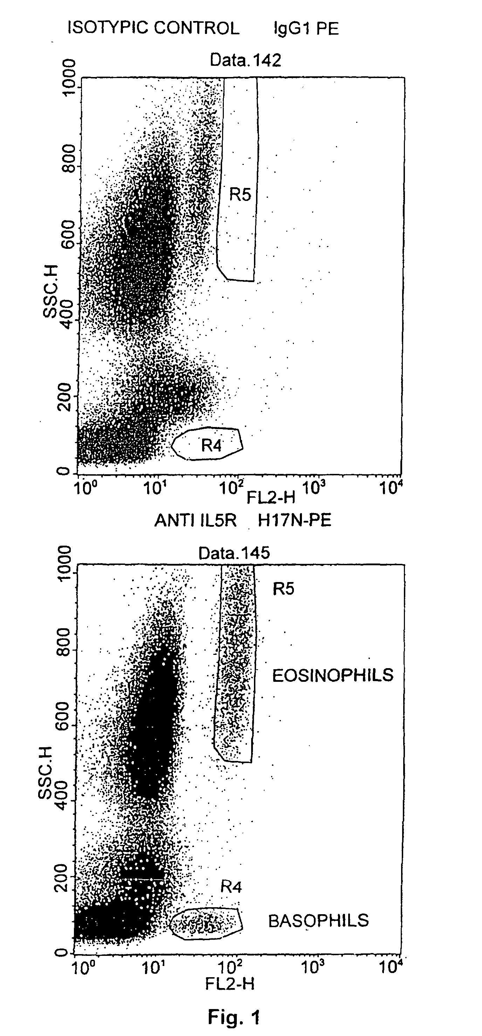

La figure 1 est l'illustration d'un "dot plot" obtenu avec l'anticorps monoclonal anti-RIL-5 H17N (B) et un contrôle isotypique (A) conjugués à la phycoérythrine. Les abscisses représentent l'intensité de fluorescence exprimée en échelle logarithmique et les ordonnées représentent la diffusion latérale.

Les polygones marqués R4 et R5 représentent respectivement les basophiles et les éosinophiles.

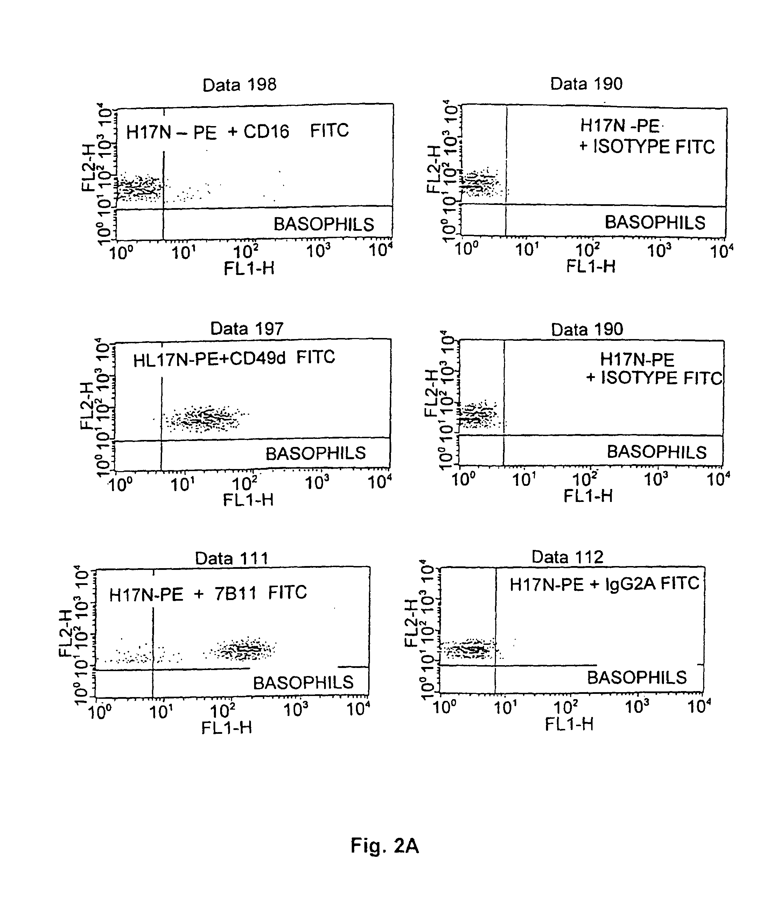

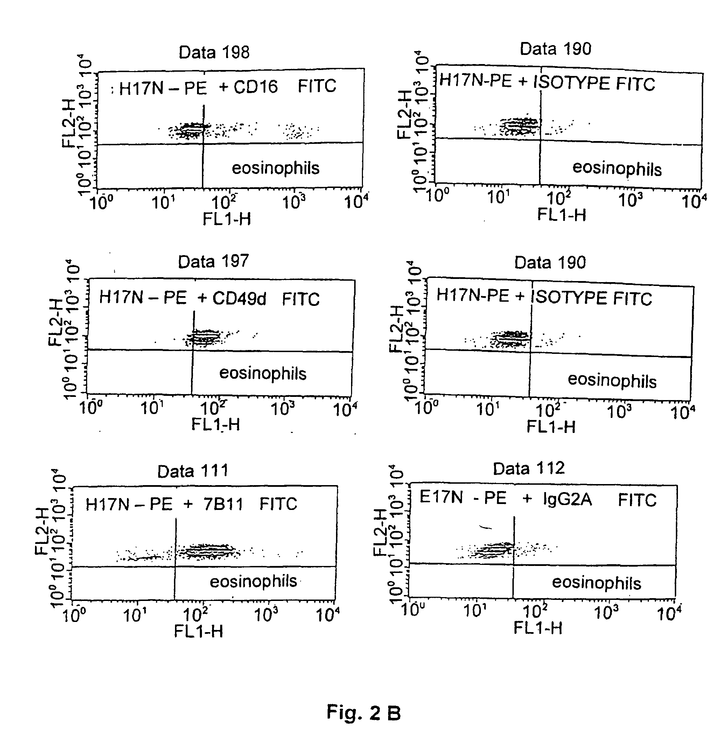

Les figures 2A et 2B sont l'illustration d'un double marquage obtenu avec l'anticorps anti-RIL-5 conjugué à la phycoérythrine pour les basophiles et les éosinophiles, respectivement. Les abscisses représentent la fluorescence émise par la fluorescéine (FITC) et les ordonnées la fluorescence émise par la phycoérythrine (PE).

Le "dot plot" de gauche représente le double marquage entre l'anti-RIL-5-PE et un anticorps spécifique d'un autre marqueur couplé au FITC. Le "dot plot" de droite représente le marquage obtenu avec un contrôle isotypique et l'anti-RIL-5-PE.

Les marqueurs utilisés sont, de haut en bas, anti-CD16 (ce marqueur est présent sur les neutrophiles, les cellules NK, les cellules de Kuppfer et une sous population des macrophages), anti-CD49d (chaîne alpha 4 des intégrines exprimée sur les basophiles et les éosinophiles mais aussi sur les lymphocytes, monocytes et thymocytes).

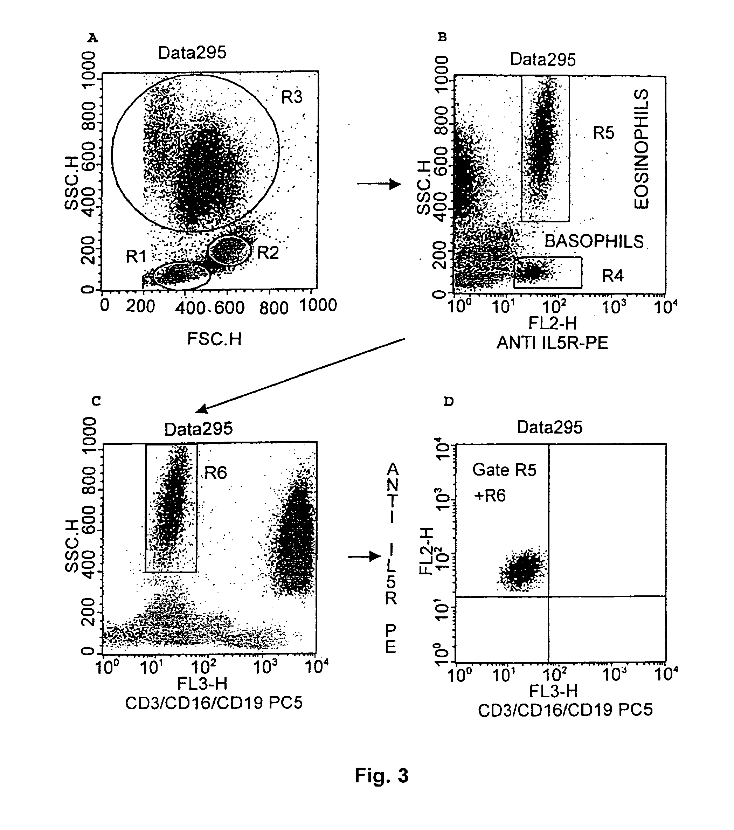

La figure 3 est l'illustration d'un double marquage obtenu avec un anticorps anti-RIL-5 conjugué à la phycoérythrine en combinaison avec un mélange d'anticorps CD3, CD19, CD16, tous conjugués à la phycoérythrine-Cyanine 5 (PECy5). Le canal FL-1 correspond à la fluorescence émise par la fluorescéine, le canal FL-2 correspond à la fluorescence émise par la phycoérythrine et le canal FL-3 correspond à la fluorescence émise par la phycoérythrine Cyanine 5.

Le diagramme A correspond à la dispersion selon la taille et la granulométrie. On distingue la région R1 qui correspond aux lymphocytes, la région R2 qui correspond aux monocytes et la région R3 qui correspond aux granulocytes.

Le diagramme B correspond à la fluorescence de l'anticorps anti-IL-5R-PE en fonction de la diffusion latérale, fonction de la taille cellulaire. R4 correspond aux basophiles et R5 correspond aux éosinophiles.

Le diagramme C représente la fluorescence du mélange d'anticorps anti-CD3, anti-CD16, anti-CD19, en fonction de la diffusion latérale. R6 correspond aux éosinophiles non marqués.

Le diagramme D représente la fluorescence du mélange d'anticorps antiCD3, anti-CD16, anti-CD19 et la fluorescence de l'anticorps anti-IL-5R-PE. Les éosinophiles se projettent dans les régions R5 et R6. Seuls les évènements présents en R5 et R6 sont pris en compte dans le diagramme D.

La figure 4 est l'illustration de la corrélation entre les pourcentages donnés par un hématimètre et le procédé de l'invention. Les abscisses représentent le pourcentage des éosinophiles (A) et le pourcentage des basophiles (B) obtenus en cytométrie de flux et les ordonnées représentent le pourcentage des éosinophiles (A) et le pourcentage des basophiles (B) donnés par l'hématimètre.

Le graphique du haut correspond aux données obtenues sur les éosinophiles et le graphique du bas aux données obtenues sur les basophiles. Les coefficients de corrélation sont données à l'intérieur de chaque graphe.

La figure 5 est l'illustration typique d'un résultat obtenu après activation des basophiles avec un anticorps anti-IgE.

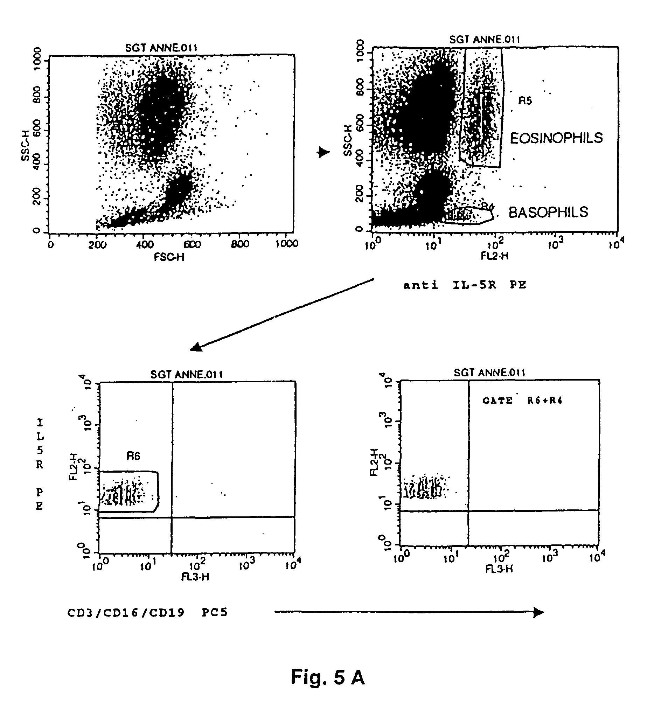

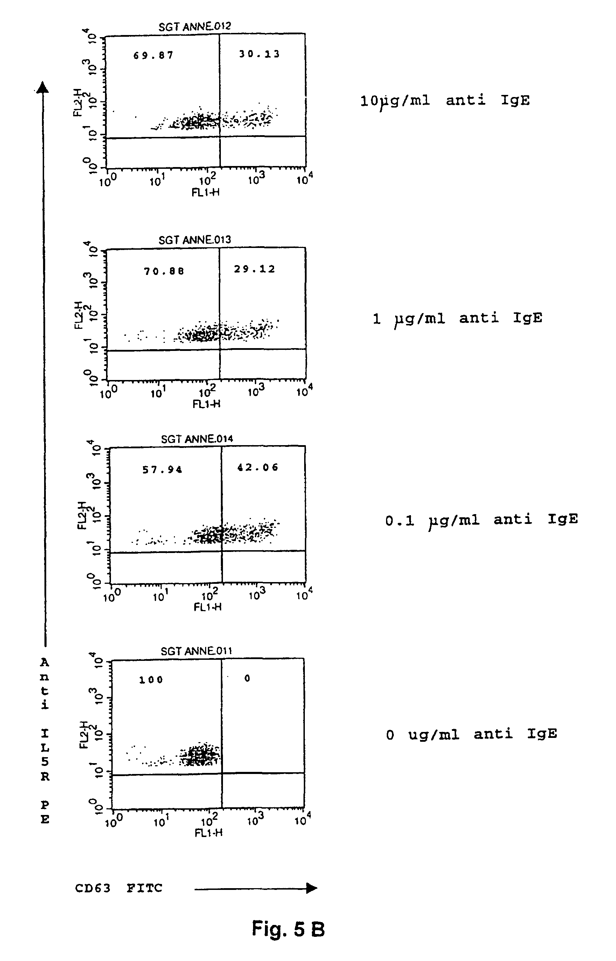

Sur cette figure 5, le "dot plot" 1 représente la taille et la granulométrie des cellules du sang, le diagramme 2 représente le marquage obtenu avec l'anticorps anti-RIL-5-PE (diagramme similaire à celui présenté sur la figure 1), le diagramme 3 représente l'isolement des basophiles et le nettoyage de la population des autres contaminants possibles, tels que les lymphocytes et les monocytes. Le diagramme 4 représente les basophiles.

La figure 5B représente 4 « dot plots » de basophiles activés. Chaque « dot plot » correspond à une stimulation de sang total par des concentrations décroissantes d'anti-IgE. Le nuage de points de chaque « dot plot » correspond à la population, isolé selon le « dot plot » 5A-4. Les abscisses représentent la fluorescence émise par la fluorescéine couplée à l'anticorps anti-CD63, et les ordonnées représentent la fluorescence émise par la phycoérythrine couplée à l'anticorps anti-RIL-5.

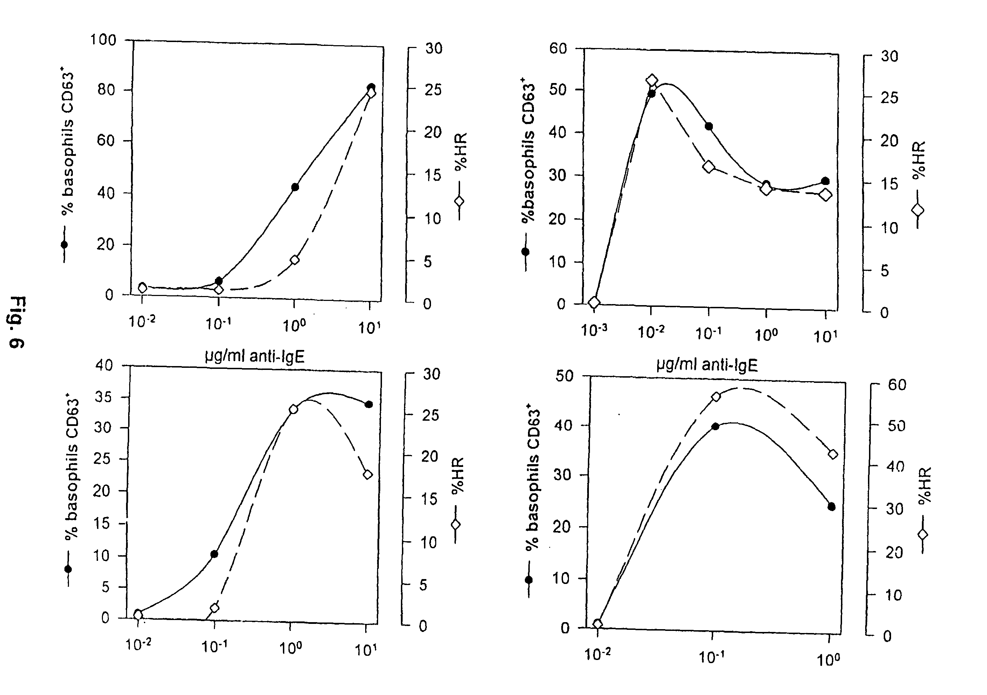

La figure 6 est l'illustration de la comparaison entre les pourcentages des cellules CD63+IL5R+ et le pourcentage d'histamine libérée.

Chaque diagramme représente un donneur. Les symboles noirs représentent le % des cellules CD63+IL-5R+ et les symboles blancs le pourcentage d'histamine libérée. Les abscisses représentent la concentration d'anti-IgE utilisée par la stimulation des basophiles exprimés en µg/ml et les ordonnées représentent le pourcentage de basophiles exprimant CD63 ou le pourcentage d'histamine libéré.

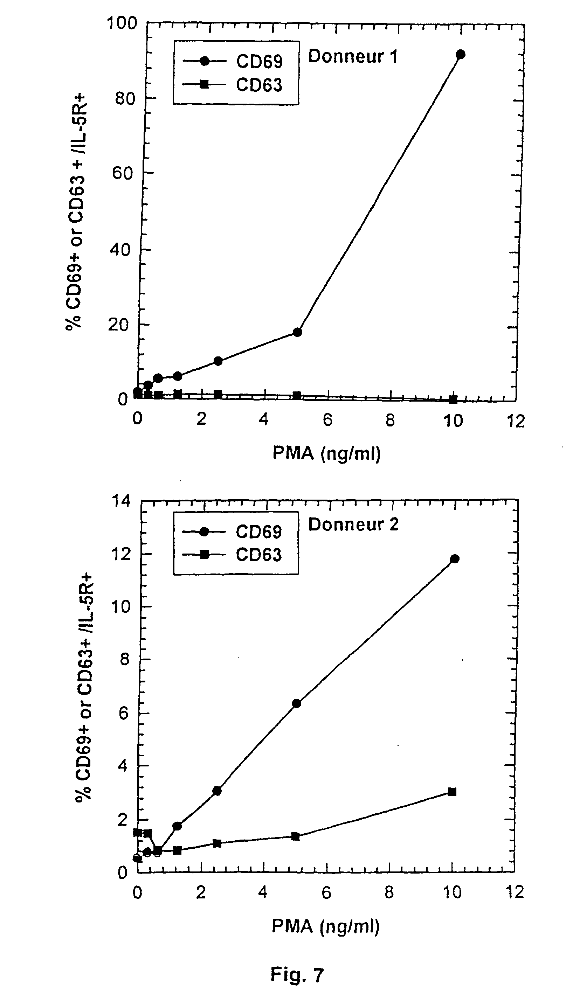

La figure 7 est l'illustration de l'activation des éosinophiles par un ester de phorbol (PMA : phorbol myristate acétate). Les abscisses représentent la concentration de PMA ajouté et les ordonnées représentent le pourcentage des éosinophiles exprimant la molécule CD69 ou CD63. Chaque diagramme représente un donneur différent.

### EXEMPLE I. Obtention des anticorps monoclonaux anti-RIL-5.

### 1- Réactifs.

Les réactifs courants (sels, tampons, etc.) ont été achetés chez Merck, Darmstadt, Allemagne. Les réactifs de culture cellulaire proviennent de Biowitthaker Virviers, Belgique et de Sigma, Saint-Louis, USA. Les anticorps monoclonaux anti-CD3, anti-CD16, anti-CD19, anti-CD49d, anti-CD63, anti-CD69 conjugués au N-isothiocyanate de fluorescéïne (FITC) ou au phycoérythrine Cyanine 5 (PECy5) et l'anticorps monoclonal anti-IgE clone E124.2.8. sont des produits commerciaux obtenus chez Immunotech, Marseille, France.

### 2- Fusion

L'anticorps anti-RIL-5, clone H17N, a été obtenu après immunisation de souris avec une forme soluble recombinante du RIL-5, protéine produite à partir d'un gène fusion RIL-5 - IL-2 et exprimée dans les cellules CHO, puis fusion des blastocytes avec les cellules de myélome X63, selon la technique classique décrite par Köhler et Milstein (1975, Nature 256, 495). La production d'anticorps a été criblée par ELISA en utilisant des plaques recouvertes avec soit la protéine hybride RIL-5-IL-2 soit avec l'IL-2. Les clones positifs sur les plaques recouvertes avec le RIL-5-IL-2 et négatifs sur les plaques recouvertes avec l'IL-2 ont été clonés par dilution limite. La sélection des anticorps s'effectue sur des cellules TF-1 en raison de leur capacité à reconnaître le RIL-5 en présence et en absence d'IL-5, critère selon la présente invention.

Parmi les anticorps monoclonaux sélectionnés, l'anticorps monoclonal anti-RIL-5, appelé H17N, présente une forte affinité pour l'antigène mais n'inhibe pas l'activité biologique de l'IL-5. En conséquence, malgré la liaison de l'IL-5 à son récepteur aucune inhibition n'est observée. L'hybridome correspondant a été déposé à la Collection Nationale de Culture de Micro-organismes (CNCM) à Paris le 3 septembre 1998 sous le N° I-2068. L'anticorps monoclonal anti-RIL-5, appelé H17N est de type IgG1.

### 3- Couplage à un marqueur fluorescent

Le couplage de l'anticorps anti-RIL-5 H17N à la phycoérythrine a été réalisé selon le protocole décrit dans « Bioconjugate techniques » par G. Hermanson, Academic Press, 1996.

### EXEMPLE 2. Analyse par cytométrie sur sang total.

### 1- Echantillons sanguins.

Les échantillons sanguins utilisés pour l'étude de corrélation ont été obtenus de l'Hôpital La Conception (Marseille, France). La formulation sanguine a été réalisée sur des tubes contenant de l'EDTA comme anticoagulant avec un appareil STKS-Coulter, Miami, USA. Le sang utilisé pour les activations des basophiles a été prélevé sur des tubes héparinés. Ces échantillons ont été prélevés sur des personnes appartenant au laboratoire.

### 2- Protocole.

L'expression du RIL-5 dans différentes sous populations cellulaires, à partir de sang total, a été analysée à l'aide d'un double immunomarquage, utilisant d'une part l'anticorps anti-RIL-5 H17N conjugué à la phycoérythrine préparé au stade 3 de l'exemple 1 et d'autre part des marqueurs spécifiques des populations cellulaires étudiées.

Le protocole utilisé est le suivant : 100 µl de sang total sont incubés avec 20 µl d'anticorps anti-RIL-5 H17N-PE et 20 µl d'un deuxième anticorps spécifique d'un autre marqueur. L'échantillon est incubé 15 min. à température ambiante.

Ensuite, 1 ml de réactif de lyse est ajouté et le mélange réactionnel vigoureusement mélangé immédiatement. Quand l'échantillon est devenu translucide (< 1 min.), 250 µl de réactif de fixation sont ajoutés et l'échantillon est à nouveau soigneusement mélangé. Trois ml de tampon phosphate isotonique (PBS 1X) sont alors ajoutés, puis l'échantillon est centrifugé pendant 3 minutes à 1200 tours par minute. Le surnageant est aspiré et 500 µl de PBS-0.5% formaldéhyde sont ajoutés. Les échantillons sont stockés à 4°C et à l'obscurité avant l'analyse au cytomètre.

La liaison non spécifique est contrôlée en utilisant un anticorps irrelevant du même isotype référence IM 0670 (Immunotech, Marseille). Ceci permet d'effectuer les réglages pertinents de l'appareil. L'analyse cytométrique des cellules marquées par les différents fluorochromes a été réalisée sur un appareil FACScalibur (Becton Dickinson, Mountain View, USA). Pour chaque échantillon, on a analysé 100 000 événements.

### 3- Identification des basophiles et des éosinophiles par cytométrie de flux.

L'anticorps anti-RIL-5 H17N a été conjugué à la phycoérythrine (PE) afin de caractériser plus facilement les cellules qui expriment le récepteur de l'IL-5. L'immunomarquage du sang total avec l'anticorps H17N-PE et un anticorps de contrôle (anticorps irrelevant de même isotype référence IM 0670) est montré sur la figure 1 (A et B).

En présence de l'anticorps de contrôle, on observe une absence de marquage (figure 1 A). Par contre, en présence de l'anticorps anti-RIL-5-PE, un décalage de la fluorescence de certaines cellules est observé (figure 1 B). Les deux nuages (fenêtres R5 et R4) des cellules marquées par l'anticorps anti-RIL-5 correspondent aux éosinophiles et basophiles. Cette observation a été confirmée grâce à plusieurs immunomarquages à l'aide d'anticorps spécifiques de différents CD's (Clusters Differentiation). Ainsi, on a observé que les cellules positives pour le RIL-5 sont négatives pour l'expression des CD3, CD19 et CD16, CDs spécifiques des lymphocytes, et négatives pour l'expression du CD14, spécifique des monocytes. Cependant, les cellules sont positives pour CD49d, protéine exprimée par les éosinophiles et les basophiles, mais aussi par les lymphocytes, monocytes et thymocytes.

Ceci démontre que les cellules marquées par l'anticorps anti-RIL-5 correspondent bien aux éosinophiles et aux basophiles.

### EXEMPLE 3. Quantification des éosinophiles et basophiles.

On a comparé la différence existant entre le pourcentage des éosinophiles et basophiles donné par l'hématimètre STKS (Coulter, Miami), utilisé à l'Hôpital de la Conception à Marseille, et le pourcentage de cellules positives pour l'expression du RIL-5, mises en évidence par l'anticorps anti-RIL-5 H17N selon l'invention.

Les basophiles et les éosinophiles sont les seuls types cellulaires marqués par l'anticorps anti-RIL-5 selon l'invention mais le nuage des basophiles est très proche du nuage de cellules correspondant aux lymphocytes.

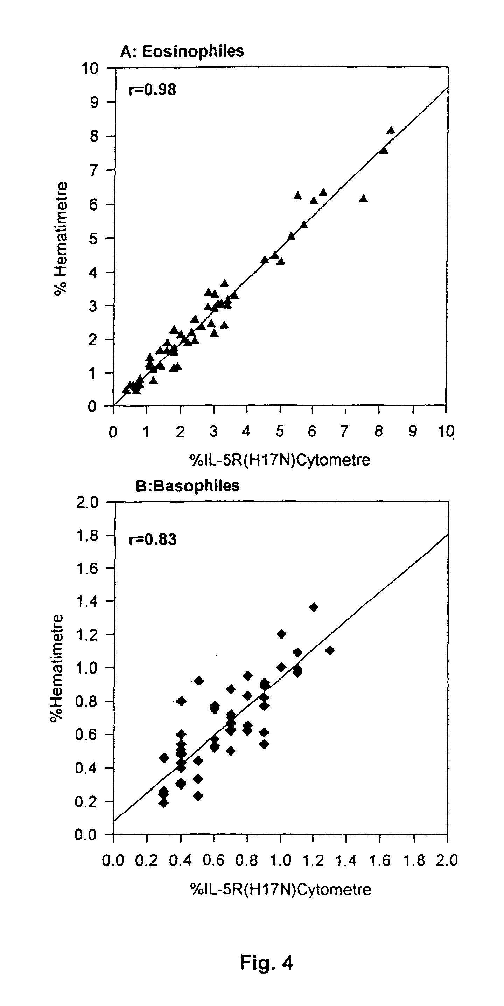

On a ajouté à l'anticorps anti-RIL-5 conjugué à la PE, un mélange d'anticorps anti-CD3, anti-CD16 et anti-CD19 conjugués au phycoérythrine Cyanine 5, ce qui permet d'exclure les évènements erratiques. Cette combinaison permet d'obtenir des valeurs plus exactes. En utilisant cette méthode, on a comparé les pourcentages d'éosinophiles et de basophiles obtenus par les deux méthodes pour 50 échantillons différents. Les résultats sont présentés dans la figure 4.

La figure 4A présente la corrélation des pourcentages d'éosinophiles obtenus avec les deux techniques. Celle-ci est de de [% Hématimètre] = 0,94 [% Cytomètre] + 0 pour n = 50. La figure 4B présente la corrélation des pourcentages de basophiles obtenus avec les deux techniques. Celle-ci est de [% Hématimètre] = 0,86 [% Cytomètre] + 0,076 pour n = 50. On observe une très bonne corrélation entre les deux méthodes, r = 0,98 et r = 0,83, démontrant la grande fiabilité du procédé selon l'invention.

### EXEMPLE 4. Activation des basophiles.

La libération d'histamine par les basophiles activés a été réalisée sur des fractions aliquotes de 300 µl de sang, déposées dans un tube et incubées en présence de différentes concentrations d'anti-IgE afin d'établir une courbe dose-réponse.

Les échantillons sont mélangés doucement et incubés à 37°C pendant 15 minutes. L'activation cellulaire est arrêtée par addition de 300 µl de tampon phosphate froid contenant 1mM d'EDTA. Les tubes sont centrifugés à +4°C durant 3 minutes à 1200 tours par minute. Le surnageant est récupéré pour quantifier l'histamine. Le culot cellulaire est remis en suspension dans 300 µl de tampon phosphate, 1mM EDTA et 100µl de cette suspension cellulaire sont analysés après immunomarquage.

La quantification de l'histamine libérée a été effectuée à l'aide d'un radio-immunodosage commercial ( Référence 1659, Immunotech, Marseille, France), suivant les instructions du fabriquant. L'histamine totale est déterminée après lyse cellulaire par dilution de 50µl de sang dans 950 µl d'eau distillée suivie de deux cycles de congélation-décongélation. L'histamine libérée après activation cellulaire est exprimée en pourcentage d'histamine totale.

L'analyse par cytométrie de flux a été réalisée après triple marquage sur 100µl de culot cellulaire repris par du PBS, avec l'anticorps anti-RIL-5 H17N-PE, l'anticorps anti-CD63-FITC et des anticorps anti-CD3, anti-CD16, anti-CD19, tous trois conjugués au PECy5. La procédure est identique à celle décrite précédemment. De la même façon, l'analyse de 100 000 cellules est acquise. Le pourcentage de cellules doublement marquées CD63+ et RIL-5+ a été déterminé après activation cellulaire.

Une expérience représentative est présentée sur la figure 5. On observe une augmentation de cellules doublement marqués (CD63 et RIL-5) en fonction de la concentration d'anti-IgE. Cette augmentation est parfaitement corrélée avec le profil d'histamine libérée comme cela est montré pour quatre donneurs différents sur la figure 6.

### EXEMPLE 5. Activation des éosinophiles

L'expression du marqueur d'activation CD69 a été réalisée sur des fractions aliquotes de 300 µl de sang, déposées dans un tube et incubées en présence de différentes concentrations de PMA (Phorbol Myristate Acetate) afin d'établir une courbe dose-réponse. Les échantillons sont mélangés doucement et incubés à 37°C pendant 6 heures. L'activation cellulaire est arrêtée en centrifugeant à +4°C durant 3 minutes à 1200 tours par minute. Le surnageant est récupéré pour quantifier l'histamine. Le culot cellulaire est remis en suspension dans 300 µl de PBS, 1 mM EDTA et 100 µl de cette suspension cellulaire sont analysés après immunomarquage.

La quantification de l'histamine libérée a été effectuée à l'aide d'un radioimmunodosage commercial (référence 1659, Immunotech, Marseille, France), suivant les instructions du fabricant. L'histamine totale est déterminée après lyse cellulaire par dilution de 50 µl de sang dans 950 µl d'eau distillée suivie de deux cycles de congélation-décongélation. L'histamine libérée après activation cellulaire est exprimée en pourcentage d'histamine totale.

L'analyse par cytométrie de flux a été réalisée après triple marquage sur 100 µl de culot cellulaire repris par du PBS, avec l'anticorps anti-RIL-5 H17N-PE, l'anticorps anti-CD69-FITC ou l'anticorps anti-CD63-FITC et les anticorps anti-CD3, anti-CD16, anti-CD19, tous trois conjugués au PECy5. La procédure est identique à celle décrite précédemment. De la même façon, l'analyse de 100.000 cellules est acquise. Le pourcentage de cellules doublement marquées CD69⁺ et RIL-5⁺ d'une part, CD63⁺ et IL-5R⁺ d'autre part a été déterminé après activation cellulaire.

Une expérience représentative est présentée sur la figure 7. On observe une augmentation de cellules doublement marquées (CD69⁺ et RIL-5⁺) en fonction de la concentration de PMA. De façon intéressante le marquage avec l'anticorps anti-CD63 sur les éosinophiles n'a pas donné de fluorescence démontrant que le CD63 est spécifique des basophiles.

### EXEMPLE 6. Kit de détection ou de quantification des éosinophiles ou des basophiles.

On a préparé un kit répondant à la composition :
- un flacon contenant un anticorps anti-RIL-5 H17N conjugué à la phycoérythrine
- un flacon contenant un mélange d'anticorps :
   Anti-CD3 conjugué au PECy5
   Anti-CD16 conjugué au PECy5
   Anti-CD19 conjugué au PECy5
- un flacon contenant un anticorps « contrôle isotypique » conjugué à la phycoérythrine
- un flacon contenant un anticorps « contrôle isotypique » conjugué au PECy5

### EXEMPLE 7. Kit de détection ou de quantification des éosinophiles ou des basophiles activés.

On a préparé un kit répondant à la composition :
- un flacon contenant un anticorps anti-RIL-5 H17N conjugué à la phycoérythrine
- un flacon contenant un mélange d'anticorps :
   Anti-CD3 conjugué au PECy5
   Anti-CD16 conjugué au PECy5
   Anti-CD19 conjugué au PECy5
- un flacon contenant un anticorps Anti-CD63 conjugué au FITC
- un flacon contenant un anticorps « contrôle isotypique » conjugué à la phycoérythrine
- un flacon contenant un anticorps « contrôle isotypique » conjugué au PECy5
- un flacon contenant un anticorps « contrôle isotypique » conjugué au FITC

### EXEMPLE 8. Kit de détection ou de quantification des éosinophiles ou des basophiles

On a préparé un kit répondant à la composition :
- un flacon contenant un anticorps anti-RIL-5 H17N conjugué à la phycoérythrine
- un flacon contenant un mélange d'anticorps :
   Anti-CD3 conjugué au PECy5
   Anti-CD16 conjugué au PECy5
   Anti-CD19 conjugué au PECy5
- un flacon contenant un anticorps « contrôle isotypique » conjugué au PECy5
- un flacon contenant du diacétate de dichlorofluorescéine

## Revendications

1. Un procédé pour la détection ou la quantification des éosinophiles et des basophiles, **caractérisé en ce qu'**il comprend la mise en contact d'un échantillon contenant éventuellement lesdits éosinophiles ou basophiles avec un anticorps monoclonal anti-récepteur de l'IL-5 chaîne alpha qui n'interfère pas avec la fixation de l'IL5 à son récepteur et qui n'inhibe pas l'activité biologique de l'IL-5 pour détecter et si désiré procéder à la quantification des éosinophiles et des basophiles.

2. Un procédé selon la revendication 1, **caractérisé en ce que** l'anticorps monoclonal anti-récepteur de l'IL-5 est un anticorps qui n'interfère pas avec les IgE

3. Un procédé selon la revendication 1 ou 2, **caractérisé en ce que** l'anticorps monoclonal anti-récepteur de l'IL-5 est un anticorps qui n'interfère pas avec l'activation cellulaire des éosinophiles ou des basophiles.

4. Un procédé selon l'une des revendications 1 à 3, **caractérisé en ce que** la détection et si désiré la quantification des éosinophiles ou des basophiles utilise un cytomètre de flux ou à balayage optique.

5. Un procédé selon l'une des revendications 1 à 4 **caractérisé en ce que** en outre l'on met en contact l'échantillon avec d'autres anticorps monoclonaux dirigés contre d'autres marqueurs des types cellulaires éosinophile ou basophile.

6. Un procédé selon la revendication 5 **caractérisé en ce que** les autres anticorps monoclonaux sont dirigés contre les marqueurs CD3, CD16 et CD19.

7. Un procédé selon l'une des revendications 1 à 6 **caractérisé en ce que** l'on procède à la détection ou la quantification des basophiles activés en mettant en outre en contact l'échantillon avec un ou plusieurs autres anticorps monoclonaux dirigés contre des marqueurs d'activation des basophiles.

8. Un procédé selon la revendication 7 **caractérisé en ce que** le marqueur d'activation est l'antigène CD 63.

9. Un procédé selon l'une des revendications 1 à 6 **caractérisé en ce que** l'on procède à la détection ou la quantification des éosinophiles activés en mettant en outre en contact l'échantillon avec un ou plusieurs autres anticorps monoclonaux dirigés contre des marqueurs d'activation des éosinophiles.

10. Un procédé de détection et de quantification des éosinophiles activés selon la revendication 9 **caractérisé en ce que** le marqueur d'activation est l'antigène CD 69.

11. Un anticorps anti-RIL-5 chaîne alpha **caractérisé par** :
- l'absence d'interférence avec la fixation de l'IL5 à son récepteur
- l'absence d'interférence avec les IgE
- l'absence d'interférence avec l'activation cellulaire des éosinophiles ou des basophiles
- l'absence d'inhibition de l'activité biologique de l'IL-5.

12. Un kit pour la détection ou la quantification des éosinophiles et des basophiles contenant
- un anticorps monoclonal anti-RIL-5 selon la revendication 11 couplé à un premier fluorochrome,
- un mélange d'anticorps marqueurs des lymphocytes, monocytes et neutrophiles, couplés à un deuxième fluorochrome.

13. Un kit pour la détection et la quantification des éosinophiles et des basophiles activés selon la revendication 12, contenant
- un anticorps monoclonal anti-RIL-5 selon la revendication 11 couplé à un premier fluorochrome,
- un mélange d'anticorps marqueurs des lymphocytes, monocytes et neutrophiles, couplés à un deuxième fluorochrome et
- des anticorps dirigés contre des marqueurs d'activation et couplés à un troisième fluorochrome.

14. Un kit pour la détection ou la quantification de l'activité oxydative des éosinophiles ou des basophiles selon la revendication 12, contenant
- un anticorps monoclonal anti-RIL-5 selon la revendication 11 couplé à un premier fluorochrome
- un mélange d'anticorps marqueurs des lymphocytes, monocytes et neutrophiles conjugués à un second fluorochrome
- un substrat marqueur de l'activité oxydative des éosinophiles ou des basophiles.

15. Un kit selon l'une des revendications 12 à 14 appliqué à l'étude de pathologies allergiques, parasitaires et leucémiques.

16. Un procédé selon l'une des revendications 1 à 10 **caractérisé en ce que** l'anticorps monoclonal anti-récepteur de l'IL-5 chaîne alpha est un anticorps de type IgG1 dont l'hybridome correspondant a été déposé à la Collection Nationale de Culture de Micro-organismes (CNCM) sous le N° I-2068.

17. Un anticorps selon la revendication 11 **caractérisé en ce que** l'anticorps monoclonal anti-récepteur de l'IL-5 chaîne alpha est un anticorps de type IgG1 dont l'hybridome correspondant a été déposé à la Collection Nationale de Culture de Micro-organismes (CNCM) sous le N° I-2068.

18. Un kit selon l'une des revendications 12 à 15 **caractérisé en ce que** l'anticorps monoclonal anti-récepteur de l'IL-5 chaîne alpha est un anticorps de type IgG1 dont l'hybridome correspondant a été déposé à la Collection Nationale de Culture de Micro-organismes (CNCM) sous le N° I-2068.

## Patentansprüche

1. Verfahren zur Detektion oder Quantifizierung von Eosinophilen und Basophilen, **dadurch gekennzeichnet, dass** es das In-Kontakt-Bringen einer gegebenenfalls Eosinophile oder Basophile enthaltenden Probe mit einem monoklonalen anti-IL-5-Rezeptor-alpha-Kette-Antikörper, der die Bindung von IL-5 an seinen Rezeptor nicht beeinflusst und der die biologische Aktivität von IL-5 bei der Detektion nicht hemmt, und gewünschtenfalls die Durchführung der Quantifizierung der Eosinophile und Basophile umfasst.

2. Verfahren nach Anspruch 1, **dadurch gekennzeichnet, dass** der monoklonale anti-IL-5-Rezeptor-Antikörper ein Antikörper ist, der keinen Einfluss auf IgE hat.

3. Verfahren nach Anspruch 1 oder 2, **dadurch gekennzeichnet, dass** der monoklonale anti-IL-5-Rezeptor-Antikörper ein Antikörper ist, der keinen Einfluss auf die Zellaktivierung von Eosinophilen oder Basophilen hat.

4. Verfahren nach einem der Ansprüche 1 bis 3, **dadurch gekennzeichnet, dass** zur Detektion und gewünschtenfalls Quantifizierung der Eosinophile oder der Basophile ein Durchfluss-Zytometer oder ein optisches Scan-Zytometer verwendet wird.

5. Verfahren nach einem der Ansprüche 1 bis 4, **dadurch gekennzeichnet, dass** die Probe weiters mit anderen monoklonalen Antikörpern in Kontakt gebracht wird, die gegen andere Marker vom eosinophilen oder basophilen Zelltyp gerichtet sind.

6. Verfahren nach Anspruch 5, **dadurch gekennzeichnet, dass** die anderen monoklonalen Antikörper gegen die Marker CD3, CD16 und CD19 gerichtet sind.

7. Verfahren nach einem der Ansprüche 1 bis 6, **dadurch gekennzeichnet, dass** die Detektion oder Quantifizierung von aktivierten Basophilen vorgenommen wird, indem die Probe weiters mit einem oder mehreren anderen monoklonalen Antikörpern in Kontakt gebracht wird, die gegen Aktivierungsmarker der Basophilen gerichtet sind.

8. Verfahren nach Anspruch 7, **dadurch gekennzeichnet, dass** der Aktivierungsmarker das Antigen CD63 ist.

9. Verfahren nach einem der Ansprüche 1 bis 6, **dadurch gekennzeichnet, dass** die Detektion oder Quantifizierung von aktivierten Eosinophilen vorgenommen wird, indem die Probe weiters mit einem oder mehreren anderen monoklonalen Antikörpern in Kontakt gebracht wird, die gegen Aktivierungsmarker der Eosinophile gerichtet sind.

10. Verfahren zur Detektion und Quantifizierung von aktivierten Eosinophilen nach Anspruch 9, **dadurch gekennzeichnet, dass** der Aktivierungsmarker das Antigen CD69 ist.

11. Anti-RIL-5-alpha-Kette-Antikörper, **gekennzeichnet durch**:
- das Fehlen jeglicher Beeinflussung der Bindung von IL-5 an seinen Rezeptor,
- das Fehlen jeglicher Beeinflussung von IgE,
- das Fehlen jeglicher Beeinflussung der Zellaktivierung von Eosinophilen oder Basophilen,
- das Fehlen jeglicher Hemmung der biologischen Aktivität von IL-5.

12. Set zur Detektion oder Quantifizierung von Eosinophilen und Basophilen, enthaltend
- einen an ein erstes Fluorochrom gekoppelten monoklonalen anti-RIL-5-Antikörper nach Anspruch 11,
- eine Mischung von an ein zweites Fluorochrom gekoppelten Marker-Antikörpern für Lymphozyten, Monozyten und Neutrophile.

13. Set zur Detektion und Quantifizierung von aktivierten Eosinophilen und Basophilen nach Anspruch 12, enthaltend
- einen an ein erstes Fluorochrom gekoppelten monoklonalen anti-RIL-5-Antikörper nach Anspruch 11,
- eine Mischung von an ein zweites Fluorochrom gekoppelten Marker-Antikörpern für Lymphozyten, Monozyten und Neutrophile,
- gegen Aktivierungsmarker gerichtete und an ein drittes Fluorochrom gekoppelte Antikörper.

14. Set zur Detektion oder Quantifizierung der oxidativen Aktivität von Eosinophilen oder Basophilen nach Anspruch 12, enthaltend
- einen an ein erstes Fluorochrom gekoppelten monoklonalen anti-RIL-5-Antikörper nach Anspruch 11,
- eine Mischung von an ein zweites Fluorochrom gekoppelten Marker-Antikörpern für Lymphozyten, Monozyten und Neutrophile,
- ein Markersubstrat für die oxidative Aktivität von Eosinophilen oder Basophilen.

15. Set nach einem der Ansprüche 12 bis 14 zur Verwendung bei der Untersuchung von allergischen, parasitären und leukämischen Erkrankungen.

16. Verfahren nach einem der Ansprüche 1 bis 10, **dadurch gekennzeichnet, dass** der monoklonale anti-IL-5-Rezeptor-alpha-Kette-Antikörper ein Antikörper vom Typ IgG1 ist, dessen entsprechendes Hybridom bei der Collection Nationale de Culture de Micro-organismes (CNCM) unter der Nummer I-2068 hinterlegt ist.

17. Antikörper nach Anspruch 11, **dadurch gekennzeichnet, dass** der monoklonale anti-IL-5-Rezeptor-alpha-Kette-Antikörper ein Antikörper vom Typ IgG1 ist, dessen entsprechendes Hybridom bei der Collection Nationale de Culture de Micro-organismes (CNCM) unter der Nummer I-2068 hinterlegt ist.

18. Set nach einem der Ansprüche 12 bis 15, **dadurch gekennzeichnet, dass** der monoklonale anti-IL-5-Rezeptor-alpha-Kette-Antikörper ein Antikörper vom Typ IgG1 ist, dessen entsprechendes Hybridom bei der Collection Nationale de Culture de Microorganismes (CNCM) unter der Nummer I-2068 hinterlegt ist.

## Claims

1. A process for the detection or quantification of eosinophils and basophils, **characterised in that** it comprises bringing a sample optionally containing said eosinophils or basophils into contact with an iL-5 anti-receptor (alpha chain) monocionai antibody which does not interfere with the fixing of IL-5 to its receptor and which does not inhibit the biological activity of IL-5 in order to detect and, if desired, to quantify the eosinophils and basophils.

2. A process according to claim 1, **characterised in that** the IL-5 anti-receptor monoclonal antibody is an antibody which does not interfere with IgE.

3. A process according to claim 1 or 2, **characterised in that** the IL-5 anti-receptor monoclonal antibody is an antibody which does not interfere with the cell activation of eosinophils or basophils.

4. A process according to one of claims 1 to 3, **characterised in that** the detection and, if desired, the quantification of eosinophils or basophils uses a flow cytometer or optical scanning cytometer.

5. A process according to one of claims 1 to 4, **characterised in that**, in addition, the sample is brought into contact with other monoclonal antibodies directed against other markers of the eosinophil or basophil cell types.

6. A process according to claim 5, **characterised in that** the other monoclonal antibodies are directed against the markers CD3, CD16 and CD19.

7. A process according to one of claims 1 to 6, **characterised in that** the detection or quantification of activated basophils is carried out by, in addition, bringing the sample into contact with one or more other monoclonal antibodies directed against basophil activation markers.

8. A process according to claim 7, **characterised in that** the activation marker is the CD63 antigen.

9. A process according to one of claims 1 to 6, **characterised in that** the detection or quantification of activated eosinophils is carried out by, in addition, bringing the sample into contact with one or more other monoclonal antibodies directed against eosinophil activation markers.

10. A process for the detection and quantification of activated eosinophils according to claim 9, **characterised in that** the activation marker is the CD69 antigen.

11. An anti-IL-5R antibody **characterised by**:
- the absence of interference with the fixing of IL-5 to its receptor,
- the absence of interference with IgE,
- the absence of interference with cell activation of eosinophils or basophils,
- the absence of inhibition of the biological activity of IL-5.

12. A kit for the detection or quantification of eosinophils and basophils containing
- an anti-IL-5R monoclonal antibody according to claim 11 conjugated to a first fluorochrome,
- a mixture of antibody markers for lymphocytes, monocytes and neutrophils, conjugated to a second fluorochrome.

13. A kit for the detection and quantification of activated eosinophils and basophils containing
- an anti-IL-5R monoclonal antibody according to claim 11 conjugated to a first fluorochrome,
- a mixture of antibody markers for lymphocytes, monocytes and neutrophils, conjugated to a second fluorochrome and
- antibodies directed against activation markers and conjugated to a third fluorochrome.

14. A kit for the detection or quantification of the oxidative activity of eosinophils or basophils containing
- an anti-IL-5R monoclonal antibody according to claim 11 conjugated to a first fluorochrome,
- a mixture of antibody markers for lymphocytes, monocytes and neutrophils, conjugated to a second fluorochrome,
- a marker substrate for the oxidative activity of eosinophils or basophils.

15. A kit according to one of claims 12 to 14 applied to the study of allergic, parasitic or leukaemic pathologies.

16. A process, antibody or kit according to one of claims 1 to 10, **characterised in that** the IL-5 anti-receptor (alpha chain) monoclonal antibody is an antibody of the IgG1 type, the corresponding hybridoma of which was deposited with the Collection Nationale de Culture de Micro-organismes (CNCM) under no. 1-2068.

17. An antibody according to claim 11 **characterised in that** the IL-5 anti-receptor (alpha chain) monoclonal antibody is an antibody of the IgG1 type, the corresponding hybridoma of which was deposited with the Collection Nationale de Culture de Micro-organismes (CNCM) under no. 1-2068.

18. A kit according to one of claims 12 to 15 **characterised in that** the IL-5 anti-receptor (alpha chain) monoclonal antibody is an antibody of the IgG1 type, the corresponding hybridoma of which was deposited with the Collection Nationale de Culture de Micro-organismes (CNCM) under no. 1-2068.
